# EUROPEAN PATENT APPLICATION

(11) **EP 2 505 217 A2**
(43) Date of publication of application: **03.10.2012**
(21) Application number: 12154798.8
(22) Date of filing: 09.02.2012
(51) Int. Cl.: A61M 1/00

(54) **Automated biological sample collection system and method**

(30) Priority: 13.02.2011 GB 201102495
(71) Applicant: Innovo Mimetics Ltd., 92583 Jerusalem (IL)
(72) Inventor: Goldstein, Ron, 92583 Jerusalem (IL); Goldstein, Robert, 92583 Jerusalem (IL); Goldstein, Jonathan, 92583 Jerusalem (IL)
(74) Representative: Harrison IP Limited

(57) **Abstract**

An automated biological sample collection system (2100) and method are disclosed. The system comprises an incubator (2120), an applicator and a sampler. The incubator is configured to control the environment of a plurality of eggs (20,22,24), the applicator is configured to deliver erogenous material to the plurality of eggs and at least one sampler is configured to collect samples from the plurality of eggs, invasively or non-invasively.

## Description

### FIELD OF THE INVENTION

The embodiments disclosed herein relate to an automated sample collection system and method. In particular a system is disclosed for performing assays on fertilized eggs.

### BACKGROUND

The embryonic chick has been used as an in vivo model for the investigation of a number of biological systems for over a century. It is noted for its simplicity, availability, immunodeficient properties, and for the highly vascularized structure of the Chorioallantoic Membrane (CAM).

Many in vivo systems, particularly model systems for studying human tissues and organs, require transplanting an examined graft into an immune compromised animal, in order to avoid graft rejection. Such animals, for example, Severe Combined Immune Deficient (SCID) mice, are expensive, sickly and hard to maintain.

The avian egg provides a low cost and readily available alternative to in vivo testing upon sentient animals. The avian egg has been used in growth of viruses for vaccine generation, angiogenesis assays, teratogenicity testing, tumor cells and the like.

Such assays may be performed on the embryos, or on microbes, cells and tissues grafted, injected or applied to fertilized avian eggs. For example, the Hen's Egg Test-Chorioallantoic Membrane (HET-CAM) assay is a well-known method for screening the irritancy potential of topically applied compositions such as cosmetics.

However, such procedures are generally performed manually, are labor intensive and are not practical on the large scale. Furthermore, because there is human intervention when handling the eggs as well as in the sampling and analysis of the assays, these procedures are not easily repeatable. It is therefore difficult to produce large numbers of identical eggs and assays to be used for statistical analysis.

It will be appreciated, therefore, that there is a need for an automated process for producing biotechnological assays. The system and method described herein address this need.

### SUMMARY OF THE EMBODIMENTS

Embodiments described herein disclose a biological sample collection system. The system comprises at least one incubator, configured to control the environment of at least one egg; at least one applicator, configured to deliver exogenous material to the at least one egg; and at least one sampler, configured to collect samples from the at least one egg. Typically, the egg comprises a fertilized avian egg. Optionally, the at least one egg comprises a chimeric system.

Where required, the system may further comprise at least one egg support apparatus for accommodating the at least one egg. The egg support apparatus may be configured to turn the at least one egg during incubation. Optionally, the egg support apparatus is configured to transport the at least one egg between a plurality of zones. Variously, the egg support apparatus comprises at least one of: an egg holder configured to hold the egg within its shell, a cup for holding a shell-less egg, a disposable cup element and an egg-turning mechanism.

Where at least one egg is a fertilized egg, the incubator may be configured to provide environmental conditions selected to support viability of the fertilized egg. Variously, the incubator may be configured to control at least one environmental parameter selected from a group consisting of: temperature, humidity, air circulation, anti-microbial agent content, pH, egg-turning, pressure, gaseous composition, oxygen content and the like as well as combinations thereof. Where appropriate, the incubator further comprises a vitality indicator configured to detect signs of life from the egg.

In some embodiments, the applicator comprises: a delivery mechanism; a manipulator configured to manipulate the delivery mechanism relative to the egg; and a feedback mechanism configured to provide feedback to the manipulator so as to control position of the delivery mechanism. Optionally, the applicator further comprises at least one reservoir for storing the exogenous material.

Variously, the delivery mechanism is selected from at least one of a group consisting of a syringe, a needle, a cantilevered needle, a pipette, a multichannel pipette, tweezers, plastic or silicon tubing, a magnetic dropper and combinations thereof. Accordingly, the applicator may be configured to deliver exogenous material selected from at least one of a group consisting of cells, tumor cells, bacteria, viruses, chemicals, drugs, skin explants and external modulators. The applicator is configured to deliver the exogenous material to at least one of a group comprising compartments of the egg, blood vessel, embryonic organs, CAM and combinations thereof.

Variously, the manipulator is selected from at least one of a group consisting of micromanipulators, robotic arms, articulated arms, telescopic arms and tracks, runners and combinations thereof. Typically, the manipulator may be configured to manipulate the egg support apparatus. Alternatively, or additionally, the manipulator may be configured to manipulate the delivery mechanism.

Variously, the feedback mechanism comprises at least one sensor selected from a group consisting of video sensors, light sensors, sonar, shadow contrast detectors, interferometers, piezoelectrical elements, tuning forks and combinations thereof.

According to certain embodiments of the system, the sampler may be configured to collect samples invasively from the egg. Variously, the sampler comprises a non-invasive imager selected from at least one of a group consisting of photographic apparatus, radioactivity detectors, MRIs, fluorescence imagers, luminescence imagers, ultrasonic imagers, heat detectors, X-ray imagers and combinations thereof.

Where required, the system may further comprise a shell removal apparatus configured to remove at least a section of the shell of the egg.

Optionally the system further comprises a controller in communication with at least one of the incubator, applicator, sampler, shell removal apparatus and egg support apparatus. The controller may be operable to control at least one factor selected from: incubation conditions, incubation periods, calculation of incubation periods, selection of incubation periods, transfer of eggs from the incubator to the applicator, transfer of eggs from the incubator to the sampler, applicator control, detection of non-viable eggs, removal of non-viable eggs, image analysis, coordination of feedback for the manipulator, shell removal, sampler activation, data collection and the like as well as combinations thereof.

In other embodiments a method is taught for collecting biological samples. The method comprises the steps of: obtaining a sample collection system comprising at least one incubator, at least one applicator and at least one sampler; obtaining at least one fertilized egg; the at least one applicator delivering at least one set of exogenous material to the at least one egg; incubating the egg; and the at least one sampler collecting at least one sample from the at least one egg. Optionally, the period of incubation is selected to be sufficient to allow engraftment of a population of cells to the chorioallantoic membrane (CAM) of the fertilized egg.

Where required, the step of the applicator delivering exogenous material may comprise: removing at least a part of the egg shell of the fertilized egg thereby exposing at least part of the chorioallantoic membrane (CAM); and placing a population of cells in contact with the CAM.

Optionally, the step of the applicator delivering exegonous material comprises administering an external modulator selected from at least one of a group consisting of a chemical agent, a biological agent, a contact sensitizer, an antigen, an allergen, a tumor-associated antigen, thermal stress, radiation, a mechanical barrier, a mechanical trauma and a gaseous agent. Variously, the external modulator may be administered in a manner selected from topical administration, subcutaneous administration, injection into the explant, injection into the explant vasculature and injection into fertilized egg vasculature.

### BRIEF DESCRIPTION OF THE FIGURES

For a better understanding of the embodiments and to show how it may be carried into effect, reference will now be made, purely by way of example, to the accompanying drawings.

With specific reference now to the drawings in detail, it is stressed that the particulars shown are by way of example and for purposes of illustrative discussion of selected embodiments only, and are presented in the cause of providing what is believed to be the most useful and readily understood description of the principles and conceptual aspects of the invention. In this regard, no attempt is made to show structural details in more detail than is necessary for a fundamental understanding; the description taken with the drawings making apparent to those skilled in the art how the several selected embodiments may be put into practice. In the accompanying drawings:
Fig. 1 is a block diagram showing the main elements of an automated biological sample collection system;
Figs. 2A and 2B are schematic cut away isometric projections representing an embodiment of a biological sample collection system;
Fig. 3A is a schematic isometric projection representing a further embodiment of a biological sample collection system;
Fig. 3B shows a possible shell removal mechanism for use in embodiments of the biological sample collection system;
Fig. 4A schematically represents a possible applicator for use with the biological sample collection system;
Fig. 4B schematically represents a possible embodiment of the delivery mechanism being used to inject exogenous material into the Chorioallantoic Membrane (CAM) of a shell-less egg; and
Fig. 5 is a flowchart representing a method for the automatic collection of biological samples from avian eggs.

### DESCRIPTION OF THE SELECTED EMBODIMENTS

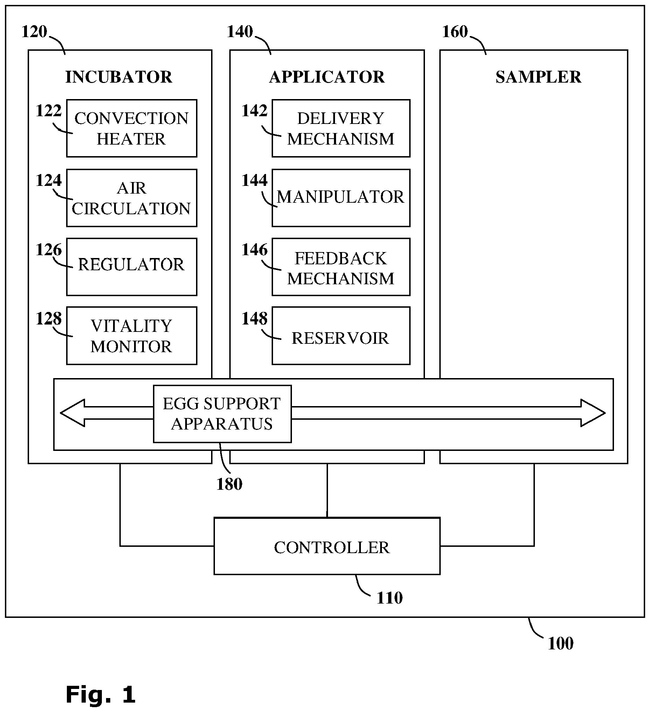

Reference is now made to the block diagram of Fig. 1 which represents the main element of an embodiment of a biological sample collection system 100. The system 100 includes an incubator 120 for incubating an egg, an applicator 140 for applying exogenous material to the egg, a sampler 160 for collecting biological samples from the egg, an egg support apparatus 180 and a controller 110 operable to coordinate the system.

It is a feature of the embodiment of the biological sample collection system 100 that it may be used to automate biological screening, particularly of fertilized avian eggs. Consequently, the system 100 may increase the throughput of biotechnological sampling thereby enabling large scale performance of biotechnological assays.

A biological sample collection system 100 of the type disclosed herein may be usefully applied to a variety of technologies. For example, the rapid sampling may make it practical to grow viruses in avian eggs which are harvested to generate vaccines.

In other applications, human or mammalian cancer cells may be transplanted to eggs. These chimeric systems may be used to perform assays of treatments such as chemotherapies, radiotherapies and the like. This technique may be used for drug development applications, for personalized medicine or the like.

Assays may be performed upon various microbes, cells, tissues, organ sections or the like. These may be injected into, grafted upon or otherwise applied to the embryo or fertilized egg. It is particularly noted that assays may be performed upon chick embryos or upon the Chorioallantoic Membrane (CAM) of the eggs.

Alternatively, or additionally, assays may be performed for angiogenic properties of substances applied to the CAM or for toxicity to the embryo (teratogenicity) or human or mammalian tissues transplanted to the CAM. Such assays may be used to examine irritation, sensitization and the like.

Still further applications will occur to the skilled practitioner.

### INCUBATOR

The incubator 120 of the biological sample collector 100 is configured to control the environmental conditions of the eggs. Typically, the incubator 120 maintains environmental conditions selected to support the viability of the fertilized eggs. Furthermore, the incubator may be adapted to control various environmental parameters such as ambient temperature, humidity, air circulation, anti-microbial agent content, ambient pH, egg-turning, pressure, gaseous composition, oxygen content and the like. The incubator may include a convection heater 122, a water reservoir, for maintaining humidity, an air circulation system 124 such as a fan, as well as regulators 126 operable to maintain conditions within selected ranges. It is further noted that the egg support apparatus 180 may be adapted to turn the eggs during incubation, for example, by tilting a tray repeatedly to simulate the motion of an egg incubated in a natural environment.

The biological sample collection system 100 may be adapted to a variety of types of eggs. Typically, chicken, turkey, ostrich, quail, duck, pheasant, grouse, or other bird eggs may be used. It is noted that while the incubation period for chick eggs is 21 days, turkey eggs have longer incubation periods of 28 days while ducks may have incubation periods of up to 42 days depending on the species. Thus the species of the host eggs may be selected to suit requirements, for example such that implanted cells are maintainable for longer.

In various embodiments, the biological sample collection system 100 may be used to collect samples from modified avian eggs wherein at least a portion of the egg shell has been removed from the fertilized avian egg. In these embodiments, the egg support apparatus 180 typically includes receptacles adapted for holding at least partially shell-less fertilized eggs. A number of references describe well-known protocols for incubating shell-less, fertilized avian eggs (Hamamichi and Nishigori 2001. Toxicol. Lett. 119: 95-102; Fisher, C. J. 1993. Chick Embryos in Shell-less Culture, Pages 105-115, in Tested studies for laboratory teaching, Volume 5 (C. A. Goldman, P. L. Hauta, M. A. O'Donnell, S. E. Andrews, and R. van der Heiden, Editors). Proceedings of the 5th Workshop/Conference of the Association for Biology Laboratory Education (ABLE); Tufan et al 2004. Akdogan Esat Adiguzel Neuroanatomy, 2004, 3 8-11). Particular reference is made to United States patent application publication number US 2009/0064349, which is incorporated herein by reference. US 2009/0064349 discloses various methods of cultivating transplanted skin explants onto the CAM of fertilized avian eggs. None of these references disclose, or suggest, an automated process for sample collection.

The ambient temperature of incubation is typically within the range of 30 to 40 degrees Celsius such that viable embryos may be maintained. The selected temperature may depend upon other requirements, for example, without wishing to be limited to any particular theory, it is particularly noted that for various applications a temperature of 40 degrees Celsius may be used to increase the effectiveness of chemical inhibitors of tumor growth while supporting the viability of the host embryo (Taylor and Williams, Biochemistry vol. 42, 1956 pp. 57-60).

According to various embodiments, a plurality of incubators may be provided, or a plurality of compartments within an incubator, such that various eggs may be subjected to different environmental conditions during incubation. Additionally or alternatively, the incubator may be adapted to provide gradual variation of environmental conditions, the effects of which may be tested.

In some embodiments, a vitality monitor 128 may be provided which is operable to monitor the eggs during incubation for signs of life from the egg. Such signs of life may include an embryonic heartbeat, pulse, blood flow, staining with and detection of vitality dyes or the like. According to one embodiment, the implanted cells may be labeled to measure proliferation by dilution, in which the count per cell decreases although the overall count remains the same. For example, a cyanine dye, such as DiI may be used for fluorescently labeling membranes, or tritiated nucleotides may be used for radioactively labeling DNA. Other labels may permanently label cells, such as viral infection with a fluorescent tag such as lentivirus expressing GFP. These labels can be measured by a vitality monitor 128 adapted to perform, among other techniques, fluorescence microscopy for DiI, GFP or the like, or MRI for tritiated probes or the like.

It will be appreciated that the vitality monitor may allow non-viable eggs to be identified early. Optionally, an egg removal system may be provided to remove such eggs from the biological collection system 100.

### APPLICATOR

The applicator 140 is configured to deliver exogenous material to the eggs. The applicator typically includes a delivery mechanism 142, a manipulator 144 and a feedback mechanism 146.

The delivery mechanism 142 is configured to deliver the exogenous material to the eggs. Exogenous material to be applied may be an implant such as a cell culture administered to the egg. For example, implants may include tumor cells, skin explants, biopsied material or the like. Such implants may be applied to fertilized eggs following an initial incubation period. Alternatively, or additionally, other exogenous material may include foreign bodies such as bacteria, viruses or external modulators.

As suits requirements, the delivery mechanism 142 may be adapted to inject the exogenous material into a compartment of the egg such as the amnion or yolk sac, or into a blood vessel of the egg, the embryo or the CAM. Additionally or alternatively, the delivery mechanism 142 may be adapted to apply the exogenous material directly onto the CAM.

It is noted that, as outlined below, in order to improve engraftment in the egg, the egg immune response may be reduced by irradiating, using X-rays, gamma rays or the like, prior to engraftment of the implanted cells. Accordingly, X-ray or gamma ray sources may be incorporated into the system, perhaps in compartments isolated by lead lining or the like. Alternatively, the system may be adapted such that where required, eggs may be removed and irradiated, perhaps automatically, as necessary. The system may be further configured to administer immunosuppressant drugs.

Accordingly, various applicators 140 may include delivery mechanisms 142 such as syringes, needles, cantilevered needle, pipettes, multichannel pipettes, tweezers, magnetic droppers or the like.

Optionally, a reservoir 148 may be provided for storing the exogenous matter before application to the egg. A reservoir may be provided including an agitator such as a stirrer or the like in order to maintain a constant composition of an agent to be delivered to the egg. Alternatively, or additionally, a reservoir may include an array of wells for containing agents of predetermined composition. For example, an 8 X 12 array of wells may contain material to be delivered by an eight channel pipette into an array of eggs. It will be appreciated that other configurations may occur to the practitioner.

According to various embodiments, external modulators may include, but are not limited to, chemical agents, biological agents, radiation, mechanical aggression, thermal stress, contact sensitizers, allergens, gaseous agents, mechanical barriers and the like.

Chemical agents may include surfactants, retinoids, carotenoids, food additives, moisturizers, mustard gas, organic molecules, inorganic compounds, vitamins, UV absorbing agents, UV protecting agents, perfumes, cosmetic formulations, lacquers, glues, paints, colorants, detergents, balms, creams, dyes, hair dyes, emulsions, gels, greases, shake lotions, pastes, oils, liposome formulations, lotions, mousses, ointments, suspensions, aqueous solutions, salves, solvents, shampoos, pollutants, steroids, shower gels, antibiotics, sulfa drugs, antiseptics, disinfectants, herbal formulations, anti-inflammatory agents, pharmaceutical compositions, aerosols, cleaning products, powders, petroleum jelly, anti-microbial agents, soaps, nail polish, acid rain, gasoline, kerosene, alcohols, industrial solvents, caustic chemicals, herbicides, metals, chelating agents, pesticides, mediations, fumigants, insecticides, fungicides, cleaning materials, contact sensitizers, allergens, impregnated dressings, solutions, occulative dressings, compression dressings,, gallium compounds, kinase modulating agents, phosphate buffered saline (PBS), enzyme inhibitors, protease, secretions of plants and animals, endotoxins, antimicrobial formulations, tazarotene formulations, bexarotene formulations, azole formulations, topical antibiotic formulations (e.g. tetracycline family), plant derived toxins (e.g. poison ivy), animal-derived toxins, putative ameliorative agents (e.g. aloe vera), depilatory or hair growth-enhancing reagents, putative anti-aging formulations and the like as well as combinations thereof.

Biological agents may include, but are not limited to, hormones (e.g. estrogens), peptides, cytokines, chemokines, interferon formulations, nucleic acids, proteins, carbohydrates, carotenoids, lipids, fatty acids, prions, enzymes, lectins, antibodies and the like as well as combinations thereof.

Following application of exogenous material, the eggs together with the exogenous material may be returned to the incubator for an additional period for continued incubation. During this second period, anastomosis of the host and implant blood vessels may occur. The implant is typically nourished by the chick's blood and gases exchanged by chick erythrocytes. It may therefore be necessary to isolate the implant from microbes in the ambient atmosphere outside of the egg shell which could contaminate the incubating implant and possibly lead to tissue degradation. Possible means for isolating the implant or sealing the egg include, but are not limited to, covering with saran wrap, adhesive tape, the egg shell itself, glass-coverslips sealed with wax, providing a sterile environment and the like.

In some embodiments, the environment provided during the second incubation period may differ from that during the initial incubation period, for example, by selecting chemical compositions or exposure to radiation which may influence the development of the implants such that environmental conditions may be tested.

The manipulator 144 is provided to manipulate the delivery mechanism 142 relative to the egg. This is necessary in order for the delivery mechanism 142 to bring the exogenous material to the desired region of the egg without damaging the egg. For example, where the delivery mechanism 142 is required to inject material into a blood vessel, the manipulator 144 is operable to direct the injection head of the delivery mechanism to the relevant blood vessel and at the required angle of attack. Similarly, where a cell culture is to be applied to the CAM, for example, the manipulator 144 is operable to bring the delivery mechanism 142 into the desired region without damaging the surrounding tissue.

Various manipulators may be utilized in embodiments of the biological sampling system 100. These include micromanipulators, robotic arms, articulated arms, telescopic arms, tracks, runners and the like as well as combinations thereof.

According to various embodiments, the manipulator 144 may be operable to act upon the delivery mechanism 142, moving it into position relative to the egg. Alternatively, the manipulator 144 may be operable to move the egg into position relative to the delivery mechanism 142, possibly by manipulating the egg support apparatus 180.

In order to control the movement of the manipulator 144, the feedback mechanism 146 is operable to provide feedback. The feedback mechanism 146 typically includes at least one sensor configured to sense the position of the delivery mechanism 142 relative to the egg, and a processor operable to direct the manipulator 144 to the desired position. Various feedback sensors may be used for such an arrangement; these include video sensors, sonar, shadow contrast detectors, interferometers, piezoelectrical elements, tuning forks and combinations thereof.

For example, a CCD camera may be linked to a feedback system to control a robotic arm manipulating the delivery system. Fine control in the approach to the target region may be supplemented by interferometric analysis of a laser beam reflected from the surface of the target region, alternatively or additionally, a piezoelectric element attached to at least one tine of a tuning fork may be used to monitor changes in the resonant frequency of the tuning fork as it approaches the surface. Other feedback mechanisms may be preferred as suit requirements.

### SHELL REMOVAL MECHANISM

In some embodiments, the system 100 may further include a shell removal mechanism. The shell removal mechanism is provided to remove at least a section of the shell in order to provide access to the CAM and the embryo within.

Where only a section of the shell is to be removed, the shell-removal mechanism may include a cutter for slicing a ring around the egg, and a sucker for removing the cut ring. Where a shell-less egg is required the shell removal mechanism may be configured to break the fertilized egg into a suitable egg support apparatus 180.

### EGG SUPPORT APPARATUS

The egg support apparatus 180 may be provided in order to hold the eggs in the sample collection system 100. Accordingly, the egg support apparatus 180 is typically a tray having an array of stands for supporting the eggs within the system 100. As noted above, where shell-less eggs are to be incubated, the egg support apparatus 180 may have suitably adapted receptacles, for example cups with covers. Optionally, such receptacles may be disposable.

According to some embodiments, the egg support apparatus may be constructed from transparent material, such as glass, PVC or the like. It will be appreciated that such a construction would allow imaging of the eggs contained by the egg support apparatus from various angles.

Apart from containing the eggs within the system 100, the egg support apparatus 180 may have additional functionality as required. For example, the egg support apparatus may be adapted to provide a turning motion to the eggs during incubation.

Typically, the egg support apparatus 180 is adapted to move between various zones of the system 100. For example, a tray supporting an array of eggs, may be mounted upon a rail and adapted to move between separate compartments or chambers for incubation, application of exogenous material and/or sampling.

Furthermore, the egg support apparatus 180 may itself form part of the manipulator 144 for positioning the egg relative to the delivery mechanism 142.

### SAMPLER

The sampler 160 is provided to collect biological samples from the eggs. Sampling may be invasive, possibly even destructive to the egg. Additionally or alternatively, non-invasive or remote sampling may be preferred.

For example, material may be removed invasively for analysis, biopsy, assay and the like. Removed material may be used in biological analysis techniques such as, but not limited to, Polymerase Chain Reaction (PCR), Fluorescence-Activated Cell Sorting (FACS), Flow Cytometry (FCM), Enzyme-Linked Immunosorbent Assay (ELISA), Enzyme-Linked Immunosorbent Spot (ELISPOT), Enzyme Multiplied Immunoassay Technique, RAST test, Radioimmunoassay, Immunohistochemistry, Immunofluorescence, dye absorption, enzymatic activity and the like. Indeed, where required, the system 100 may incorporate any or all of these material analysis devices.

Alternatively, samples may be obtained non-invasively using imaging techniques such as photographic imaging, radiographic detection, Magnetic Resonance Imaging (MRI), fluorescence imaging, luminescence imaging, ultrasonic imaging, heat detection, x-ray imaging, scintigraphy, Positron Emission Tomography (PET), photoacoustic imaging, thermography, and the like as well as combinations thereof. Indeed, where required, the system 100 may incorporate any or all of these non-invasive sampling devices.

It will be appreciated that non-invasive sampling may be useful, particularly where ongoing monitoring is required. For example, the sampler 160 may be configured to periodically take measurements from the egg during incubation.

Still another method of sampling may be to install a catheter or similar device into a blood vessel or one of the liquid compartments with a valve that can be opened, in order to take multiple samples. For example, a needle attached to fine silicon tubing may be inserted into a CAM vessel, with a valve that can be opened for sampling blood at desired intervals.

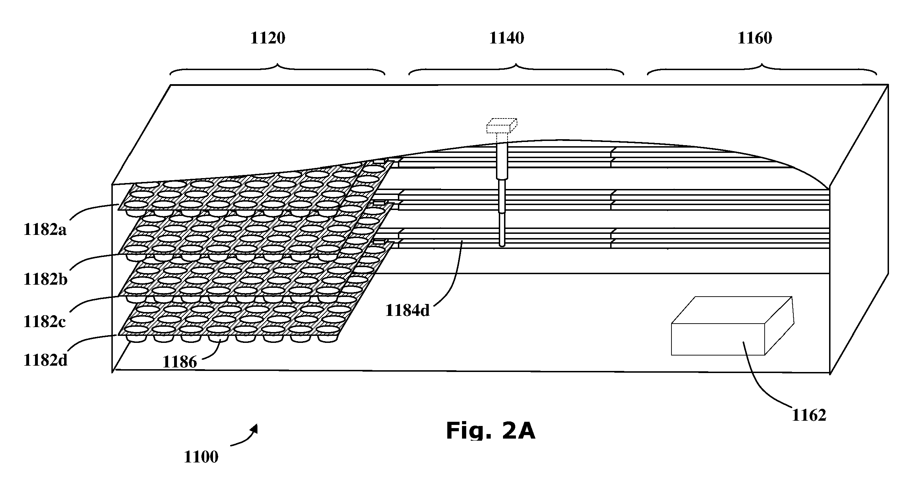

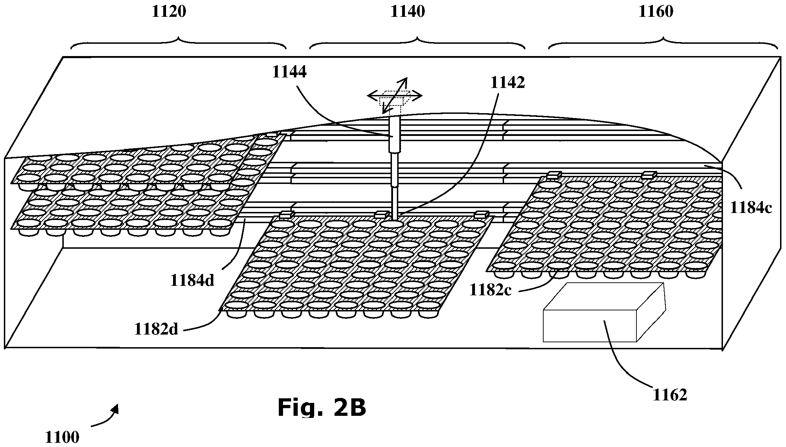

In order to further illustrate the various embodiments of the system, reference is now made to Figs. 2A and 2B which show schematic cut away isometric projections representing one embodiment of a biological sample collection system 1100.

The embodiment includes an incubation chamber 1120, an application chamber 1140 and a sampling chamber 1160. The egg support mechanism 1180 [add to fig] includes multiple trays 1182a-d mounted upon tracks 1184a-d.

The egg support trays 1182a-d are provided to contain fertilized eggs. In the example provided in Figs. 2A and 2B, each tray includes a 9 X 8 array of egg receptacles1186. Where appropriate, the receptacles may be configured to contain complete eggs, shell-less eggs or partially shell-less eggs. It will be appreciated that the 9 X 8 array of the example may be of particular use where an eight channel syringe is used to apply exogenous material to the eggs, for example, when such matter is applied manually. Other configurations may be preferred as suit requirements.

The trays 1182a-d are mounted on tracks 1184a-d which extend through all three chambers 1120, 1140, 1160 and along which the trays may be moved. With particular reference now to Fig. 2A, showing the system 1100 during one of the incubation phases, all the trays 1182a-d are arranged in the incubation chamber 1120.

The incubator is configured to keep the incubated eggs at a temperature of approximately between 30 to 40 degrees Celsius so as to maintain an environment suitable for the continued viability of fertilized eggs. It will be appreciated that a turning mechanism (not shown) may be provided to periodically tilt the trays to simulate the turning of eggs during natural incubation.

Referring now to Fig. 2B, the system 1100 is shown in an alternative configuration; the one tray 1182d has been transferred, along its associated track 1184d, to the application chamber 1140, for introduction of exogenous material to the eggs and another tray 1182c has been transferred to the sampling chamber 1160.

In the application chamber 1140, a delivery mechanism 1142 mounted to a manipulator 1144 may be directed to a selected egg within the tray 1182d.

The manipulator 1144 of the example includes a roof mounted telescopic arm configured to move the delivery mechanism 1142 to the desired position within the application chamber. It will be appreciated that other manipulators may be additionally or alternatively provided as required.

The tray 1182c is shown transferred to the sampling chamber 1160 wherein it may be inspected using an imager 1162. Various imagers 1162 may be utilized in the system 1100 as outlined herein. Additionally or alternatively, material may be collected from the eggs for analysis.

In order to prevent movement of the embryo during application of the exogenous material, the application chamber 1140 may be cooled to a low temperature such as around 18 degrees Celsius or so. Accordingly, an insulating partition (not shown) may be provided between the incubation chamber 1120 and the application chamber 1140.

The application chamber 1140 may be adapted for other methods for preventing movement such as applying gentle suction to the membrane or dropping sterile filter paper rings onto the CAM to maintain stretched blood vessels for injection/removal of blood.

Following application of the exogenous material, the tray 1182d may be returned to the incubation chamber for additional periods of incubation. The process may be repeated a plurality of times with different exogenous materials being applied. For example, an incubated egg may be transferred to the application chamber 1140 initially for application of an implant or the like. Then, the egg may be returned to the incubation chamber 1120 for further incubation until the engraftment of the implant occurs. The egg may be returned to the application chamber 1140, following re-incubation so that an external modulator, such as described above, may be applied to the egg. The egg may be transferred again to the incubation chamber 1120 for further incubation during which the egg may be periodically transferred to the sampling chamber 1160 for examination.

It will be appreciated that the three chambered system 1100 described hereinabove in relation to the embodiment of Figs. 2A and 2B represents only an example of a possible biological sampling system. Alternatively, other embodiments may be preferred to suit different requirements.

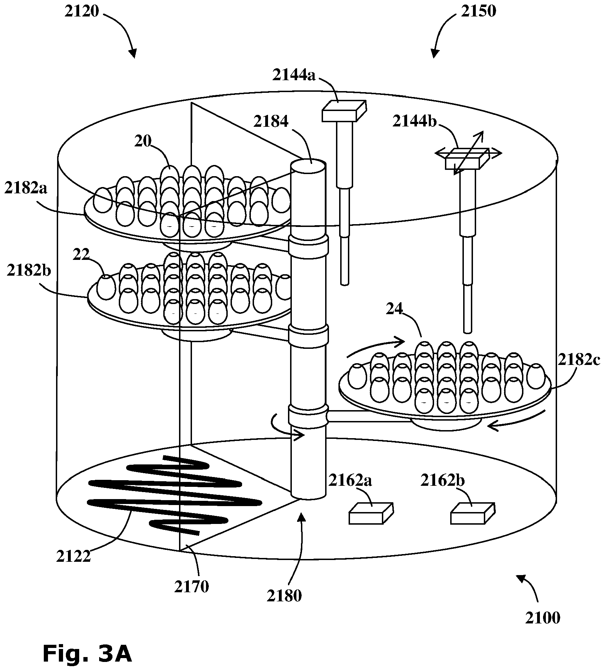

Referring now to Fig. 3A, there is shown a schematic isometric projection representing a further embodiment of a biological sample collection system 2100. The system 2100 of the embodiment of Fig. 3A includes an incubator compartment 2120, a second compartment 2150 and an egg support apparatus 2180.

The incubator compartment 2120 has a convection heater 2122 and an air circulator or filtration system or the like. The second compartment 2150 is a multifunctional space which may include various mechanisms such as manipulators 2144a, 2144b, samplers 2162a 2162b, shell removal mechanisms (not shown) and the like. Partitions 2170 may be provided to divide the separate compartments; such partitions may be insulated and/or sealed to allow the separate compartments 2120, 2150 to support different environmental conditions.

The egg support apparatus 2180 of the embodiment of Fig. 3A includes multiple egg trays 2182a-c coupled to a central support column 2184 in a manner that allows rotation. The trays 2182a-c are configured to individually rotate around the central support column 2184 such that each tray may be transferred from the incubator compartment 2120 to the second compartment 2150. Optionally, the trays 2182a-c may be further configured to rotate about their central axes to position the eggs 20 supported thereby.

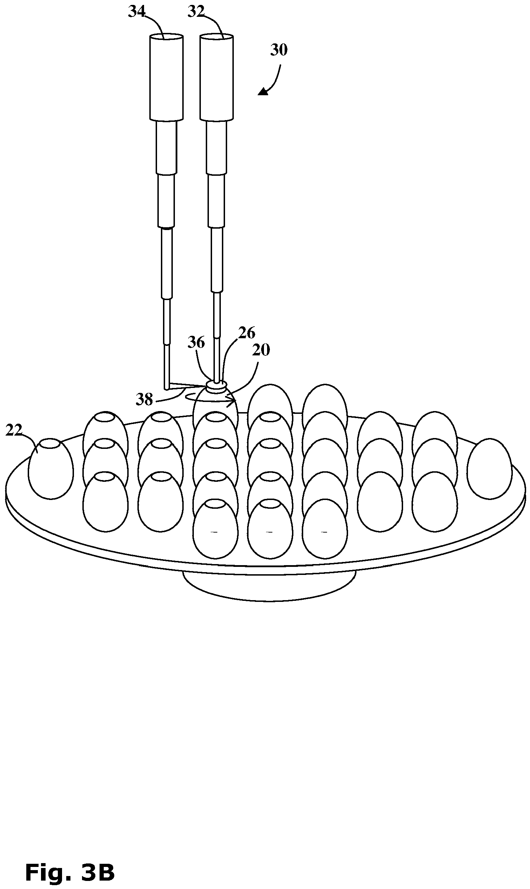

In the example, a first tray 2182a is shown supporting complete eggs 20 during an incubation phase. Following an initial incubation period, the tray may be transferred to the second chamber 2150 where a shell removal mechanism, such as described herein below in relation to Fig. 3B, may be used to remove a section of the egg shell, and the applicator may be used to apply exogenous material to the egg. A second tray 2182b is shown supporting eggs 22 which have had the top section of their shells removed, typically, such eggs may be returned to the incubator for a second incubation period following the application of exogenous material.

The third tray 2182c is shown supporting eggs 24 following a second incubation period after which the eggs 24 have been transferred into the second chamber 2150 for application of additional exogenous material, for example, an external modulator such as described hereinabove or the like.

Referring now to Fig. 3B, a possible shell removal mechanism 30 for use with embodiments of the biological sample collection system is shown. The shell removal mechanism 30 includes two manipulators 32, 34. The first manipulator 32 has a suction head 36 operable to engage the top section 26 of the egg shell of an egg 20. The second manipulator has a blade head 38 configured to sever the top section 26 from the egg 20. Optionally, each egg may be rotatable individually to further manipulate the egg's position. Other shell removal mechanisms 30 are known and may occur to the skilled practitioner.

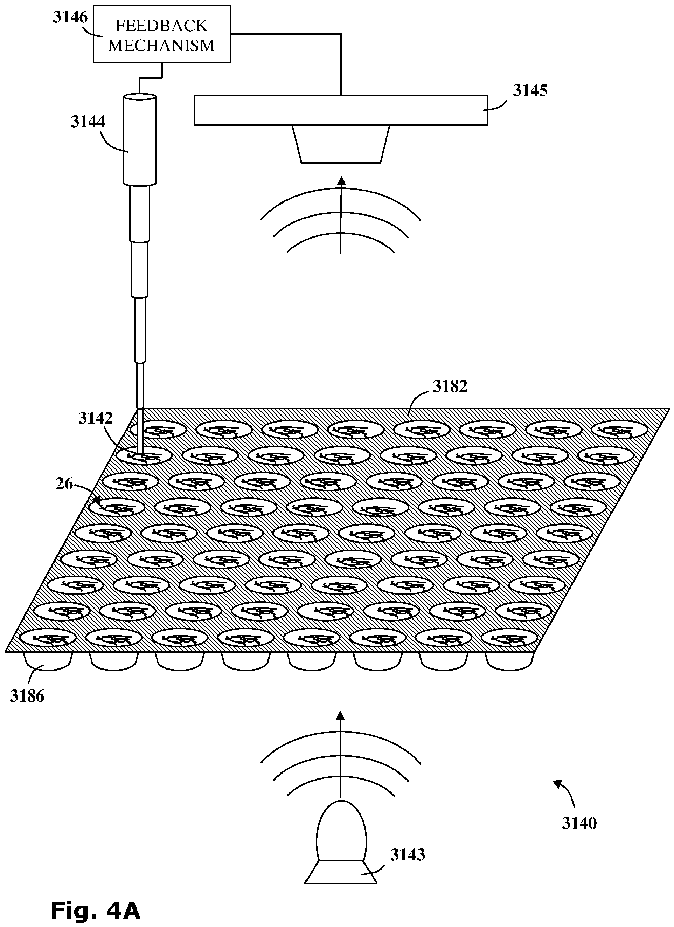

Referring now to Fig. 4A, a schematic representation is shown of a possible applicator 3140 for use with a biological sample collection system and a tray 3182 of receptacles for containing shell-less eggs. The applicator 3140 includes a delivery mechanism 3142, a manipulator 3144, an illuminator 3143, a detector 3145 and a feedback mechanism 3146.

The tray 3182 contains egg receptacles 3186. The egg receptacles 3186 are preferably made of a material transparent to the type of radiation emitted by the illuminator 3143 such that the detector 3145 is able to monitor the relative positions of the delivery mechanism 3142, the organs of the shell-less eggs and their embryos contained within the receptacles 3186.

The detector 3145 is typically in communication with the feedback mechanism 3146 which is operable to direct the delivery head 3142 accordingly.

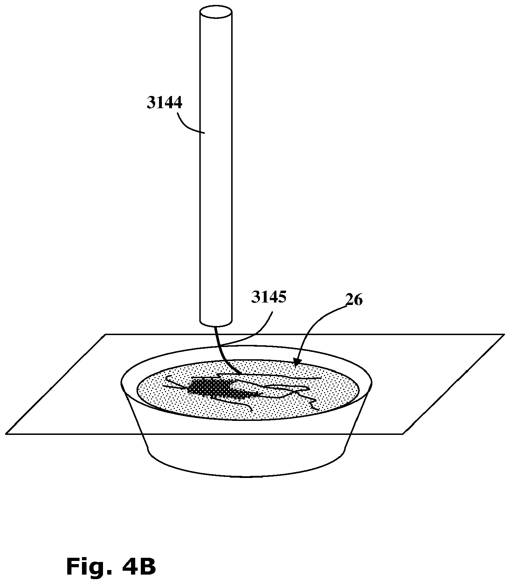

Referring now to Fig. 4B a possible embodiment of the delivery mechanism 3142 is shown. The delivery mechanism 3142 includes an angled needle 3145 which may be used to inject exogenous material into the Chorioallantoic Membrane (CAM) of a shell-less egg 26 or other organs or blood vessels.

### CONTROLLER

Referring back to the block diagram of Fig. 1, in order to control and coordinate the biological sample collection system 100, a controller 110 may be provided. The controller 110, which may be in communication with the various functional elements, may be operable to perform a sequence of steps according to an algorithm.

Accordingly, the controller 110 may be functional to coordinate steps such as incubation conditions, incubation periods, calculation of incubation periods, selection of incubation periods, transfer of eggs from the incubator to the applicator and/or sampler, applicator control, detection of non-viable eggs, removal of non-viable eggs, image analysis, coordination of feedback for the manipulator, shell removal, sampler activation, data collection and the like.

Where appropriate the controller 110 may include a processor, a database, a memory and/or a user interface as required.

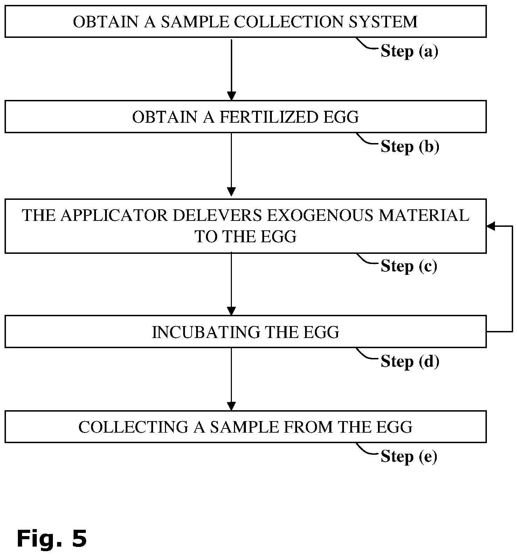

Referring now to the flowchart of Fig. 5, the steps are presented of a possible method for use with the system for the collection of biological samples. The method includes the steps:
Step (a) - Obtaining a sample collection system such as described herein;
Step (b) - Obtaining at least one fertilized egg;
Step (c) - An applicator delivering exogenous material to the egg;
Step (d) - Incubating the egg; and
Step (e) - Collecting at least one sample from the egg.

Where appropriate, step (c) may be further subdivided into the steps:
- removing at least a part of the egg shell of the fertilized egg thereby exposing at least part of the chorioallantoic membrane (CAM); and
- placing a population of cells in contact with the CAM.

It is noted that step(c) and step (d) may be repeated a plurality of times, for example, where a variety of types of exogenous material are to be applied. Thus, where required, a population of cells may be applied to the CAM of a fertilized egg and the egg then returned to the incubator for a period of time sufficient to allow engraftment of the implant to occur. The applicator may then be used to deliver another exogenous material such as a chemical or biological agent before again incubating the egg.

Where appropriate, the method may further include the steps of removing a part of the egg shell of the fertilized egg, or removing the whole egg shell, thereby exposing and providing access to the chorioallantoic membrane (CAM). The modulator may be administered using a variety of techniques, such as topical administration, subcutaneous administration, injection into the explant, injection into the explant vasculature, injection into fertilized egg vasculature and the like, as well as combinations thereof.

It is further noted that, in order to improve engraftment in the egg, the egg immune response may be reduced by irradiating, using X-rays, gamma rays or the like, prior to engraftment of the implanted cells. Such irradiation may inhibit the normal xenograft rejection of an intact host immune system. Furthermore, for engraftment of blood and bone marrow malignancies, irradiation may make hematopoietic niches available to absorb the donor cells in the host bone marrow. It is noted that such irradiation typically leaves the egg host free of compounds that may interfere with the growth of the graft in the therapeutic conditions that are being tested. Additionally or alternatively, other immunosuppressant drugs such as cyclosporine or the like may be used to improve engraftment due to immunosuppression of the host rejection response, as well as the anti-apoptotic effect that may limit graft cell death.

### EXAMPLES

For illustrative purposes only, a selection of examples is presented below to demonstrate the usefulness of various embodiments of the biological sample collection system 100:

### Growth of Viruses for Generation, Testing or Development of Vaccines

Viruses may be produced in embryonated chick eggs after injection into the allantois, blood supply, yolk sac, or chorion. By using a biological sample collection system such as described hereinabove, the accuracy of the injection may be increased. This is particularly so when shell-less eggs are incubated. Furthermore, it is noted that the use of shell-less eggs also allows for rapid and accurate identification of dead embryos which may be readily removed.

Accordingly, eggs may be broken into individual compartments of the egg support apparatus, optionally this step may be performed by the shell-removal mechanism, although alternatively this step may be carried out manually. The eggs may be incubated to the appropriate age for the particular virus and administration route.

Once the embryos reach the appropriate age, the egg support apparatus may move the eggs to the applicator where, using machine vision, the appropriate portion of the egg (allantois, amniotic sac, yolk sac, blood vessel) may be identified, and injected robotically, for example, using an automated version of a syringe-holding device such as that described by Kelling and Schipper, 1976.

For example, the yolk or appropriate blood vessels may be identified by means of their color, shape, size or other chemical, physical or biological characteristics, and the syringe may thereby be guided towards them automatically. Although the allantois and amniotic sac may be more difficult to identify automatically, an automated algorithm may be generated in order to allow identification of such elements.

Alternatively, an operator using a video-link to the inside of the machine may perform the initial positioning of the manipulator, to allow the injection to proceed automatically thereafter.

While still in the chamber, dead embryos, or those sufficiently growth-stunted to be likely to die may be readily identified and removed.

### Assays of Chemotherapies on Human or Mammalian Cancer Cells Transplanted to the Egg

### A) Hematological malignancies:

### i) Patient samples of blood cancer for theranostics.

According to another application, blood cancer samples may be collected from individual patients for theranostics. A suspension of blood cancer cells taken from the peripheral blood or bone marrow of patients suffering from candidate conditions, such as leukemia, lymphoma, myeloma and the like, may be prepared for injecting into eggs.

Fertilized shell-less eggs may be prepared in individual compartments, either manually or using a shell removal apparatus. The shell-less eggs may be incubated to the appropriate age for each type of virus and administration route.

Upon the eggs reaching sufficient maturity, typically after 7-9 days of incubation for chicken eggs or 8-11 days for turkey eggs, the tray of the egg support mechanism may be moved to the applicator.

Using machine vision, appropriate blood vessels in the eggs may be identified, by color and size, for example. The manipulator may guide a syringe toward the blood vessels. The syringe would typically be filled with the prepared suspension of blood cancer cells with typically about 1-2 million cells being injected into each egg.

Following the application of cancerous cells, the tray of the egg support apparatus may be returned to the incubator for an additional 2-3 days during which the cancer may become engrafted.

Following the engraftment of the cancer, the applicator may be used to inject chemotherapeutic agents intravenously.

Following a third incubation period, the bone marrow or other other embryo tissues such as liver, spleen or the like, may be assayed, either manually or using the sampler, for the presence of human cancer cells. Variously, sampling may use the polymerase chain reaction (PCR) for human DNA, or human cell-surface markers by fluorescence aided cell sorting, or human cell death markers. Whole animal imaging may use cells made fluorescent in the infrared with lentiviral vectors driving expression of appropriate proteins such as Turbo635 (Evrogen).

### ii) Blood cell lines for drug development.

Laboratory cancer cell lines may be obtained which are engineered to express far-red fluorescing proteins such as m-cherry, Turbo635 (Evrogen) or the like.

As above, fertilized shell-less eggs may be prepared in individual compartments, either manually or using a shell removal apparatus. The shell-less eggs may be incubated to the appropriate age for each type of virus and administration route.

Upon the eggs reaching sufficient maturity, typically after 7-9 days of incubation for chicken eggs or 8-11 days for turkey eggs, the tray of a the egg support mechanism may be moved to the applicator.

Using machine vision, appropriate blood vessels in the eggs may be identified, by color and size, for example. The manipulator may guide a syringe toward the blood vessels. The syringe would typically be filled with the cancer cell lines of blood cancer cells with typically about 1-2 million cells being injected into each egg.

Following the application of cancerous cells, the tray of the egg support apparatus may be returned to the incubator for an additional 2-3 days during which the cancer may become engrafted.

Following the engraftment of the cancer, the applicator may be used to inject chemotherapeutic agents intravenously.

Following a third incubation period, the sampler may use whole-body imaging with green exciting light to assay the killing of cancer cells by therapeutic agents.

Alternatively, non-engineered cells may be used, and cells tagged with magnetic nanoparticles such that the sampler may use MRI imaging.

### B) Solid cancers

### i) Patient samples for Theranostics

Tumor tissue may be obtained from patients by removal during surgery or biopsy, for example. Tissue may be cut into 1 mm cubes using a Mcilwain tissue chopper, for example. Alternatively, tissue may be minced and suspended in a gel such as matrigel, and applied to the CAM.

The biological sampling system may be utilized to prepare suitable host eggs, for example, using the methods described above.

The applicator may be used to place the tumor tissue cubes onto the CAM of E8 chicken embryos, for example. The eggs may be incubated again for about three days until engraftment of the tissue occurs.

Following the engraftment of the cancer, the applicator may be used to inject chemotherapeutic agents intravenously. Lipid soluble chemotherapeutic agents can be applied to the CAM without injection. Phototherapy activated-drugs (based on porphyrins) can also be used on CAM-grafted tumors.

Following a third incubation period of, say, 3-5 days, grafted tumor pieces in treated and untreated embryos may be photographed, and their size compared. Thus candidate drugs may be compared for their effectiveness in reducing tumor growth.

### ii) Cell lines for drug development.

Laboratory cancer cell lines may be obtained which are engineered to express green fluorescent protein, for example. A suspension may be prepared of such cells in a gelling matrix material (e.g. matrigel or puramatrix).

The biological sampling system may be utilized to prepare suitable host eggs, for example, using the methods described above.

The applicator may be used to place the cell suspension upon the CAM of E8 chick embryos. The suspension may be contained upon the CAM within a plastic ring, for example. The eggs may be incubated again for about three days until engraftment of the tissue occurs.

Following the engraftment of the cancer, the applicator may be used to inject chemotherapeutic agents intravenously.

Following a third incubation period of, say, 3-5 days or so, grafted tumor pieces of the laboratory line cancer cells in treated and untreated embryos may be photographed, and their size compared. Thus candidate drugs may be compared for their effectiveness in reducing tumor growth.

### iii) Metastasis studies

The biological sampling system may be used to detect metastasis of applied cancer cells to the internal organs of an embryo, using whole-animal imaging techniques such as MRI, whole animal fluorescence imaging, or dissection and histological or FACS analysis, for example, as described above.

### Assays for Angiogenic Properties of Substances Applied to the Chorioallantoic Membrane (CAM)

Preparations of angiogenic or anti-angiogenic solutions may be absorbed onto 1 cm radius pieces of filter paper, for example. The applicator of the biological sampling system described herein may be used to place such preparations onto the CAM of E5 embryos, for example.

Following an incubation period of about 2-7 days, the sampler may be used to measure the generation or reduction of blood vessels, for example, by using photography of the CAM and automated image analysis.

### Assays of Toxicity to the Embryo (Teratogenicity), Transplanted Human or Mammalian Skin Transplanted to the CAM, Including Irritation, Sensitization etc.

### i) Teratogenicity testing

New compounds for use in industrial, medical, cosmetic or other industries would be injected intravascular as above, and embryos may be examined at E18 or earlier for morphological abnormalities.

### ii) Dermatological testing.

Human skin, consisting of epidermis and a few millimeters of dermis may be obtained (i.e. from cosmetic surgery clinics), and cut into 6 mm rings with a dermal punch. The tissue may be placed dermis side down on the CAM of E6-9 eggs, and further incubated for two days until engraftment. Optionally, the CAM can be mildly abraded with lens tissue to cause bleeding and thereby improve engraftment of the skin. The cutting of the skin and the application of the samples may be performed automatically by the applicator or manually, as required.

Following engraftment, the skin may be treated with potential irritants or sensitizers, UV irradiated, treated with suntan lotion etc. and incubated for an additional time.

Following further incubation, the skin may be assayed by histology etc, using conventional methods. It is noted that the sampler may further assay the skin implant during the incubation process, or at the end of such process, using a non-invasive imaging system.

Additional dermatological applications may include testing for the introduction of material such as: viruses (herpes), genes (gene therapy), examination of transcutaneous absorption (by taking blood samples from the embryo), bacteria into the skin (acne) or the like and combinations thereof.

The scope of the disclosed subject matter is defined by the appended claims and includes both combinations and sub combinations of the various features described hereinabove as well as variations and modifications thereof, which would occur to persons skilled in the art upon reading the foregoing description.

In the claims, the word "comprise", and variations thereof such as "comprises", "comprising" and the like indicate that the components listed are included, but not generally to the exclusion of other components.

## Claims

1. A biological sample collection system comprising:
at least one incubator configured to control the environment of at least one egg;
at least one applicator configured to deliver exogenous material to said at least one egg; and
at least one sampler configured to collect samples from said at least one egg.

2. The system of claim 1 further comprising at least one egg support apparatus for accommodating said at least one egg.

3. The system of claim 2 wherein said egg support apparatus is configured to turn said at least one egg during incubation.

4. The system of claim 2 wherein said egg support apparatus is configured to transfer said at least one egg between a plurality of zones.

5. The system of claim 2 wherein said egg support apparatus comprises at least one of a group consisting of:
an egg holder configured to hold said at least one egg within its shell,
a cup for holding a shell-less egg,
a disposable cup element, and
an egg-turning mechanism.

6. The system of claim 1 wherein said incubator is configured to provide environmental conditions selected to support viability of at least one fertilized egg.

7. The system of claim 1 wherein said incubator is configured to provide environmental conditions selected to support viability of at least one chimeric system comprising a fertilized egg and an implanted cell culture.

8. The system of claim 1 wherein said incubator is configured to control at least one environmental parameter selected from a group consisting of: temperature, humidity, air circulation, anti-microbial agent content, ambient pH, egg-turning, pressure, gaseous composition, oxygen content and combinations thereof.

9. The system of claim 1 wherein said incubator further comprises a vitality indicator configured to detect signs of life from at least one fertilized egg.

10. The system of claim 1 further comprising a shell removal apparatus configured to remove at least a section of the shell of said at least one egg.

11. The system of claim 1 wherein said applicator comprises:
at least one delivery mechanism;
at least one manipulator configured to manipulate said at least one delivery mechanism relative to said at least one egg; and
at least one feedback mechanism configured to provide feedback to said at least one manipulator so as to control the position of said at least one delivery mechanism.

12. The system of claim 11 wherein said applicator further comprises at least one reservoir for storing said exogenous material.

13. The system of claim 11 wherein said at least one delivery mechanism comprises at least one of a group consisting of: syringes, needles, angled needles, cantilevered needles, pipettes, multichannel pipettes, tweezers, tubing, magnetic droppers and combinations thereof.

14. The system of claim 11 wherein said applicator is configured to deliver said exogenous material selected from at least one of a group consisting of: cells, tumor cells, bacteria, viruses, chemicals, drugs, skin explants and external modulators.

15. The system of claim 11 wherein said applicator is configured to deliver said exogenous material to at least one of a group comprising: compartments of said egg, amnion, yolk sack, blood vessels, embryonic organs, chorioallantroic membrane (CAM) of said at least one egg and combinations thereof.

16. The system of claim 11 wherein said manipulator is selected from at least one of a group consisting of:
micromanipulators, robotic arms, articulated arms,
rotatable arms, telescopic arms, tracks, runners and
combinations thereof.

17. The system of claim 11 wherein said manipulator is configured to manipulate at least one of an egg support apparatus, said at least one delivery mechanism, and a shell removal mechanism.

18. The system of claim 11 wherein said at least one feedback mechanism comprises at least one sensor selected from a group consisting of: video sensors, light sensors, sonar, shadow contrast detectors, interferometers, piezoelectrical elements, a tuning fork and combinations thereof.

19. The system of claim 1 wherein said sampler is configured to collect samples invasively from said at least one egg.

20. The system of claim 1 wherein said sampler comprises a non-invasive imager selected from at least one of a group consisting of: photographic apparatus, radioactivity detectors, MRIs, fluorescence imagers, luminescence imagers, ultrasonic imagers, heat detectors, X-ray imagers and combinations thereof.

21. The system of claim 1 further comprising a controller in communication with at least one of the incubator, applicator, sampler, shell removal apparatus and egg support apparatus, said controller operable to control at least one factor selected from: incubation conditions, incubation periods, calculation of incubation periods, selection of incubation periods, transfer of eggs from the incubator to the applicator, transfer of eggs from the incubator to the sampler, applicator control, detection of non-viable eggs, removal of non-viable eggs, image analysis, coordination of feedback for the manipulator, shell removal, sampler activation, data collection and combinations thereof.

22. A method of collecting biological samples comprising the steps:
obtaining a sample collection system comprising at least one incubator, at least one applicator and at least one sampler;
obtaining at least one fertilized egg;
said at least one applicator delivering at least one exogenous material to said at least one egg;
incubating said at least one egg; and
said at least one sampler collecting at least one sample from said at least one egg.

23. The method of claim 22 wherein the period of incubation is selected to be sufficient to allow engraftment of a population of cells to the chorioallantoic membrane (CAM) of said at least one fertilized egg.

24. The method of claim 22 wherein the step of said applicator delivering exogenous material comprises:
removing at least a part of the egg shell of said at least one fertilized egg thereby exposing at least part of the chorioallantoic membrane (CAM); and
placing a population of cells in contact with the CAM.

25. The method of claim 22 wherein the step of said applicator delivering exogenous material comprises administering an external modulator selected from at least one of a group consisting of: a chemical agent, a biological agent, a contact sensitizer, an antigen, an allergen, a tumor-associated antigen, thermal stress, radiation, a mechanical barrier, a mechanical trauma and a gaseous agent.

26. The method of claim 25 wherein said external modulator is administered in a manner selected from topical administration, subcutaneous administration, injection into the explant, injection into the explant vasculature and injection into fertilized egg vasculature.
